# EUROPEAN PATENT SPECIFICATION

(11) **EP 1 062 351 B1**
(45) Date of publication and mention of the grant of the patent: **10.05.2006**
(21) Application number: 99907615.1
(22) Date of filing: 09.03.1999
(51) Int. Cl.: C12N 15/82, C12N 9/64, C12N 5/10, C12Q 1/68, A01H 5/00

(54) **NOVEL PLANT PLASTID PROMOTER SEQUENCE**
PFLANZLICHE PROMOTORSEQUENZ AUS PLASTIDEN
SEQUENCE DE PROMOTEUR PLASTIDIAL DE PLANTE

(30) Priority: 11.03.1998 US 38878
(43) Date of publication of application: 27.12.2000
(73) Proprietor: Syngenta Participations AG, 4058 Basel (CH)
(72) Inventor: HEIFETZ, Peter, Bernard, San Diego, CA 92131 (US)
(74) Representative: Gaal, Jozsef Christopher
(86) International application number: PCT/EP1999/001515
(87) International publication number: WO 1999/046394

(56) References cited:
- WO-A-97/32011
- WO-A-97/32977
- WO-A-98/55595
- MANEN J -F ET AL: "THE ATPB AND RBCL PROMOTERS IN PLASTID DNAS OF A WIDE DICOT RANGE" JOURNAL OF MOLECULAR EVOLUTION, vol. 38, no. 6, 1 June 1994, pages 577-582, XP000617945
- KOOP, H-U., ET AL.: "integration of foreign sequences into the tobacco plastome via polyethylene glycol-mediated protoplast transformation" PLANTA, vol. 199, 1996, pages 193-201, XP002106134
- SVAB,Z. AND MALIGA, P.: "high-frequency plastid transformation in tobacco by selection for a chimeric aadA gene" PROCEEDINGS OF THE NATIONAL ACADEMY OF SCIENCES OF THE USA, vol. 90, February 1993, pages 913-917, XP002106110
- SHANKLIN, J., ET AL. : "the stroma of higher plant plastis contain ClpP and ClpC, functional homologs of Escherichia coli ClpP and ClpA: an archetypal two-component ATP-dependent protease" THE PLANT CELL, vol. 7, October 1995, pages 1713-1722, XP002106111 cited in the application
- SRIRAMAN,P., ET AL.: "the phage-type PclpP-53 promoter comprises sequences downstream of the transcription inititaion site" EMBL SEQUENCE DATA LIBRARY, 19 November 1998, XP002106112
- SRIRAMAN, P., ET AL.: "the phage-type PclpP-53 promoter comprises sequences downstream of the transcription inititaion site" NUCLEIC ACID RESEARCH, vol. 26, no. 21, November 1998, pages 4874-4879, XP002106113
- SRIRAMAN, P., ET AL. : "transcription from heterologous rRNA operon promoters in chloroplasts reveals requirement for specific activating factors" PLANT PHYSIOLOGY, vol. 117, no. 4, August 1998, pages 1495-1499, XP002106114

### SEQUENCE LISTING

<110> Novartis AG
<120> NOVEL PLANT PLASTID PROMOTER SEQUENCE
<130> PH/5-30422/CGC 1988
<140>
   <141>
<160> 12
<170> PatentIn Ver. 2.0
<210> 1
   <211> 499
   <212> DNA
   <213> Arabidopsis thaliana
<220>
   <221> misc_feature
   <222> (497)..(499)
   <223> clpP ATG start codon
<220>
   <221> misc_feature
   <222> Complement ((6)..(8))
   <223> psbB ATG start codon
<400> 1
   aaacccatgg aaatacccct ttatcaacga aaaatagaca ctatgtaact ttattgcatt 60
   ggaaaaaact atgctacgta cccccccttt ttaggtaatt atttcggaga aaggattaat 120
   atttgttcta ttctgttagt aataatggaa caattcaatt catagaaaaa aagggaagcg 180
   gatctattct atatccgata agtaccaata tgcaatgggg gttaatccta ttttctatga 240
   accaagatag ctattgttgt tgatcattca gaagcccgtt cgtaaaaaat ttcctttagt 300
   tttattcatt ctctcttact ttacttttat tttatatttt attttagctt attcaactta 360
   tgtattaaat atcattaatt taaatatgat taataaagta ggaaaaaagg ataatagtat 420
   taaaaaacga aaccccaatt ttacgtttcc acatcaaagt gaaatagaga acttcattct 480
   cttttttttt catttcatg                                              499
<210> 2
   <211> 28
   <212> DNA
   <213> Artificial Sequence
<220>
   <223> Description of Artificial Sequence: A_clpP PCR primer derived from FSTP_3982
<400> 2
   aagggacttt tggaacgcca ataggcat                                    28
<210> 3
   <211> 25
   <212> DNA
   <213> Artificial Sequence
<220>
   <223> Description of Artificial Sequence: A_psbB PCR primer derived from conserved DNA sequences around the psbB start codon
<400> 3
   cacgatacca aggcaaaccc atgga 25
<210> 4
   <211> 30
   <212> DNA
   <213> Artificial Sequence
<220>
   <223> Description of Artificial Sequence: Primer Aclp_pla
<400> 4
   gcggaattca tcattcagaa gcccgttcgt 30
<210> 5
   <211> 28
   <212> DNA
   <213> Artificial Sequence
<220>
   <223> Description of Artificial Sequence: Primer Aclp_P2b
<400> 5
   gcgtcatgaa atgaaagaaa aagagaat 28
<210> 6
   <211> 30
   <212> DNA
   <213> Artificial Sequence
<220>
   <223> Description of Artificial Sequence: Primer rps16P_1a
<400> 6
   gcgtctagat caaccgaaat tcaattaagg 30
<210> 7
   <211> 27
   <212> DNA
   <213> Artificial Sequence
<220>
   <223> Description of Artificial Sequence: Primer rps16_1b
<400> 7
   cgcaagcttc aatggaagca atgataa 27
<210> 8
   <211> 20
   <212> DNA.
   <213> Artificial Sequence
<220>
   <223> Description of Artificial Sequence: top strand primer
<400> 8
   cagttcgagc ctgattatcc 20
<210> 9
   <211> 20
   <212> DNA
   <213> Artificial Sequence
<220>
   <223> Description of Artificial Sequence: bottom strand primer
<400> 9
   gttcttacgc gttactcacc 20
<210> 10
   <211> 369
   <212> DNA
   <213> Arabidopsis thaliana
<400> 10
   cagttcgagc ctgattatcc ctaaacccaa tgaatgtgag tttttctatt ttgacttgct 60
   ccctcgctgt gatcgaataa gaatggataa gaggctcgtg ggattgacgt gagggggtag 120
   gggtagctat atttctggga gcgaactcca tgcgaatatg aagcgcatgg atacaagtta 180
   tgacttggaa tgaaagacaa ttccgaatca gctttgtcta cgaagaagga agctataagt 240
   aatgcaacta tgaatctcat ggagagttcg atcctggctc aggatgaacg ctggcggcat 300
   gcttaacaca tgcaagtcgg acgggaagtg gtgtttccag tggcggacgg gtgagtaacg 360
   cgtaagaac 369
<210> 11
   <211> 32
   <212> DNA
   <213> Artificial Sequence
<220>
   <223> Description of Artificial Sequence: top strand primer
<400> 11
   gccggaattc tcgctgtgat cgaataagaa tg 32
<210> 12
   <211>55
   <212> DNA
   <213> Artificial Sequence
<220>
   <223> Description of Artificial Sequence: bottom strand primer
<400> 12
   gccttcatga atccctccct acaactatcc aggcgcttca gattcgcctg gagtt 55

## Description

The present invention generally pertains to plant molecular biology and more particularly pertains to a novel plastid promoter isolated from *Arabidopsis thaliana* and methods of use therefor. The present invention also pertains to a novel method for utilizing protein-coding regions of plastid genes to isolate intervening regulatory sequences. The present invention further pertains to the use of novel plastid promoter sequences to improve plastid transformation efficiency.

Plastid transformation, in which genes are inserted by homologous recombination into all of the several thousand copies of the circular plastid genome present in each plant cell, takes advantage of the enormous copy number advantage over nuclear-expressed genes to permit expression levels that may exceed 10% of the total soluble plant protein. In addition, plastid transformation is desirable because plastid-encoded traits are not pollen transmissable; hence, potential risks of inadvertent transgene escape to wild relatives of transgenic plants are obviated. Other advantages of plastid transformation include the feasibility of simultaneous expression of multiple genes as a polycistronic unit and the elimination of positional effects and gene silencing that may result following nuclear transformation. Plastid transformation technology is extensively described in U.S. Patent Nos. 5,451,513, 5,545,817, 5,545,818, and 5,576,198; in Intl. Application No WO 95/16783; and in Boynton *et al., Methods in Enzymology* 217: 510-536 (1993), Svab *et al., Proc. Natl. Acad. Sci. USA* 90: 913-917 (1993), and McBride *et al., Proc. Natl. Acad. Sci. USA* 91: 7301-7305 (1994).

The basic technique for tobacco plastic transformation involves the particle bombardment of leaf tissue with regions of cloned plastid DNA flanking a selectable marker, such as an antibiotic resistance gene. The 1 to 1.5 kb flanking regions, termed targeting sequences, facilitate homologous recombination with the plastid genome and thus allow the replacement or modification of specific regions of the 156 kb tobacco plastome. Initially, point mutations in the chloroplast 16S rRNA and rps12 genes conferring resistance to spectinomycin and/or streptomycin were utilized as selectable markers for transformation (Svab ef al., *Proc. Natl. Acad. Sci. USA* 87: 8526-8530 (1990); Staub, J. M., and Maliga, P., *Plant Cell 4:* 39-45 (1992)). This resulted in stable homoplasmic transformants at a frequency of approximately one per 100 bombardments of target leaves. The presence of cloning sites between these markers allowed creation of a plastid targeting vector for introduction of foreign genes (Staub, J.M., and Maliga, P., *EMBO J.* 12: 601-606 (1993)). Substantial increases in transformation frequency were obtained by replacement of the recessive rDNA or r-protein antibiotic resistance genes with a dominant selectable marker, the bacterial aadA gene encoding the spectinomycin-detoxifying enzyme aminoglycoside-3'-adenyltransferase (Svab *et al.,* 1993). Previously, this marker had been used successfully for high-frequency transformation of the plastid genome of the green alga *Chlamydomonas reinhardtii* (Goldschmidt-Clermont, M., *Nucl. Acids Res.* 19: 4083-4089 (1991)). Techniques have also been described for the transfection of plastids in plant protoplasts (O'Neill *et al., Plant Journal* 3(5): 729-738 (1993) and Koop *et al., Planta* 199: 193-201 (1996)).

An especially preferred plant plastid promoter for use in plastid targeting vectors to express foreign genes in the plant plastid is the *clpP* gene promoter. The *clpP* gene encodes the proteolytic subunit of the Clp ATP-dependent protease, which in *Arabidopsis* is constitutively expressed in the plastids of photosynthetic and nonphotosynthetic plant tissues (Shanklin et al., *The Plant Cell* 7: 1713-1722 (1995)). *clpP* is also one of the few plant plastid genes that is retained in the genomes of non-photosynthetic plants (e.g. *Epifagus virginiana;* Morden et al. *EMBO J* 10: 3281-3288 (1991)) and the *clpP* message is known to be expressed in the plastids of the barley mutant *albostrians,* which lacks detectable plastid translational activity (Hübschmann and Bömer, Plant Mol. Biol. 36: 493-496 (1998)). Hence, the *clpP* promoter is likely to be active transcriptionally even in nongreen plastids. The characterization of the promoter region from the tobacco *clpP* gene is described in WO97/06250. In this reference, the tobacco *clpP* gene is characterized as having 5' promoter sequences that are recognized by both a nuclear encoded plastid (NEP) RNA polymerase and a plastid encoded plastid (PEP) RNA polymerase. A primary transcript arising from the tobacco *clpP* promoter sequence mapping to the -53 nucleotide position (upstream from the ATG translation initiation codon) is characterized in WO 97/06250 as being highly expressed in the bleached plastids of tobacco mutants lacking a plastid-encoded RNA polymerase by virtue of deletion of the *rpoB* gene.

A tobacco *clpP* promoter sequence has been used to drive expression of a herbicide-resistant form of the *Arabidopsis* Protoporphyrinogen IX ("PROTOX") gene in the plastids of tobacco (WO 97/32011). Identical constructs substituting a GUS reporter gene have been introduced into tobacco plastids, demonstrating that *clpP-*driven expression is not restricted to green plastids but is also found in root plastids (leucoplasts, amyloplasts) and flower plastids (chromoplasts).

Manen et al. (*Journal of Molecular Evolution* 38: 577-582 (1994)) describes the identification of the rbcL and atpB gene promoters in spinach and tobacco. WO98/55595 describes the expression of recombinant genes in plastids in both green and non-green plant tissues via novel regulatory elements, including NEP promoters.

Despite the promise shown by plastid transformation, only recently has this technology been applied to plants other than tobacco. International Application No. WO97/32977 describes methods and compositions for creating transplastomic plants in the *Cruciferae* family, such as *Brassica* and *Arabidopsis,* using leaf and cotyledon cells. However, what is also needed are novel plastid promoter sequences from plants other than tobacco, particularly *Arabidopsis,* which can be used to drive the expression of transgenes in green and non-green plastids of *Arabidopsis* and any other plant species.

In view of the above, one object of the invention is to provide a novel plastid promoter from *Arabidopsis thaliana* that is functional in all plastid types. Another object of the invention is to provide a method for utilizing protein-coding regions of plastid genes to isolate novel intervening regulatory sequences, such as novel promoter sequences or untranslated 3' or 5' RNA sequences. Still another object of the invention is to use novel plastid promoter sequences to improve plastid transformation efficiency by reducing undesired homologous recombination between native DNA sequences in the plastid genome and exogenous DNA sequences contained in chimeric DNA fragments incorporated into plastid transformation vectors.

In furtherance of these and other objects, the present disclosure describe a nucleic acid promoter isolated from the 5' flanking region upstream of the coding sequence of the *Arabidopsis* plastid *clpP* gene. In a preferred embodiment, the nucleic acid promoter is substantially similar to a promoter sequence downstream of nucleotide number 263 of SEQ. ID NO:1. In a more preferred embodiment, the nucleic acid promoter has sequence identity with a promoter sequence downstream of nucleotide number 263 of SEQ ID NO:1. In still another embodiment, the nucleic acid promoter is substantially similar to SEQ ID NO:1. In yet another embodiment, the nucleic acid promoter is comprised within SEQ ID NO:1.. In still another embodiment, the nucleic acid promoter comprises a 20 base pair nucleotide portion identical in sequence to a consecutive 20 base pair nucleotide portion of SEQ lD NO: 1. The present disclosure also encompasses a chimeric gene comprising the nucleic acid promoter operatively linked to the coding sequence of a gene of interest; a plant transformation vector comprising such a chimeric gene; and a transgenic plant, plant cell, plant seed, plant tissue, or plant plastid, each comprising such a chimeric gene.

In another aspect, the present disclosure describes a novel method for isolating intervening regulatory DNA sequences from between the protein-coding regions of two plastid genes, comprising the steps of:
(a) determining the relative orientation and either a degenerate or a specific nucleotide sequence of protein-coding regions of two plastid genes;
(b) designing a first degenerate or specific PCR primer based on the determined sequence of the protein-coding region of one of the two plastid genes;
(c) designing a second degenerate or specific PCR primer based on the determined sequence of the protein-coding region of the other of the two plastid genes;
(d) amplifying a DNA fragment using the primers of steps (b) and (c), whereby the amplified DNA fragment comprises an intervening regulatory DNA sequence from between the protein-coding regions of the two plastid genes.
In a preferred embodiment of this method, the two plastid genes are a *clpP* gene and a *psbB* gene. According to this embodiment, the intervening regulatory DNA sequence comprises a *clpP* promoter. In another preferred embodiment of this method, the two plastid genes are a 16S rRNA gene and a valine tRNA gene. According to this embodiment, the intervening regulatory DNA sequence comprises a 16S rRNA promoter.

According to the present invention there is provided an improved plastid transformation method, comprising transforming a plastid of a host plant species with a chimeric gene comprising a plastid-active regulatory sequence of an *Arabidopsis* plastid - *clpP* gene operatively linked to a coding sequence of interest, wherein the regulatory sequence has a nucleotide sequence that is less than approximately 90% identical to any native plastid *clpP* gene regulatory sequence in the host plant plastid but still functions as a plastid-active regulatory sequence, whereby undesired somatic recombination between the regulatory sequence in the chimeric gene and the corresponding native regulatory sequence in the host plant plastid is reduced. In a preferred embodiment of this method, the chimeric gene is isolated from the plastid genome of the host plant species and at least approximately 10% of the nucleotides of the regulatory sequence have been mutated. In another preferred embodiment of this method, the regulatory sequence in the chimeric gene is isolated from the plastid genome of a different plant species than the host plant species. For example, the regulatory sequence in the chimeric gene may be isolated from the plastid genome of *Arabidopsis.* In one especially preferred embodiment, the regulatory sequence in the chimeric gene is a nucleic acid promoter isolated from the 5' flanking region upstream of the coding sequence of the *Arabidopsis clpP* gene. In another especially preferred embodiment, the regulatory sequence in the chimeric gene is a nucleic acid promoter isolated from the 5' flanking region upstream of the coding sequence of the *Arabidopsis* 16S rRNA gene.

Other objects and advantages of the present invention will become apparent to those skilled in the art from a study of the following description of the invention and non-limiting examples.

### DESCRIPTION OF THE SEQUENCES IN THE SEQUENCE LISTING

SEQ ID NO:1 is the nucleotide sequence of the *Arabidopsis clpP* gene promoter region.

SEQ ID NO:2 is primer A_clpP used in Example 1.

SEQ ID NO:3 is primer A_psbB used in Example 1.

SEQ ID NO:4 is primer Adp_P1a used in Example 2.

SEQ ID NO:5 is primer Aclp_P2b used in Example 2.

SEQ ID NO:6 is primer rps16P_1a used in Example 2.

SEQ ID NO:7 is primer rps16P_1b used in Example 2.

SEQ ID NO:8 is the top-strand primer used in Example 3.

SECQ ID NO:9 is a bottom-strand primer used in Example 3.

SEQ ID NO:10 is the nucleotide sequence of the *Arabidopsis* 16S rRNA gene promoter region.

SEQ ID NO:11 is a top-strand primer used in Example 4.

SEQ ID NO:12 is a bottom-strand primer used in Example 4.

### DEFINITIONS

For clarity, certain terms used in the specification are defined and presented as follows:

Associated With / Operatively Linked: refers to two nucleic acid sequences that are related physically or functionally. For example, a promoter or regulatory DNA sequence is said to be "associated with" a DNA sequence that codes for an RNA or a protein if the two sequences are operatively linked, or situated such that the regulator DNA sequence will affect the expression level of the coding or structural DNA sequence.

Chimeric Gene / Fusion Sequence: a recombinant nucleic acid sequence in which a promoter or regulatory nucleic acid sequence is operatively linked to, or associated with, a nucleic acid sequence that codes for an mRNA or which is expressed as a protein, such that the regulator nucleic acid sequence is able to regulate transcription or expression of the associated nucleic acid sequence. The regulator nucleic acid sequence of the chimeric gene is not normally operatively linked to the associated nucleic acid sequence as found in nature.

Coding Sequence: nucleic acid sequence that is transcribed into RNA such as mRNA, rRNA, tRNA, snRNA, sense RNA or antisense RNA. Preferably the RNA is then translated in an organism to produce a protein.

Gene a defined region that is located within a genome and that besides the aforementioned coding sequence, comprises other, primarily regulatory, sequences responsible for the control of the expression, that is to say the transcription and translation, of the coding portion. A gene may also comprise other 5' and 3' untranslated sequences and termination sequences. Further elements that may be present are, for example, introns.

Gene of Interest: any gene that, when transferred to a plant, confers upon the plant a desired characteristic such as antibiotic resistance, virus resistance, insect resistance, disease resistance, or resistance to other pests, herbicide tolerance, improved nutritional value, improved performance in an industrial process or altered reproductive capability. The "gene of interest" may also be one that is transferred to plants for the production of commercially valuable enzymes or metabolites in the plant

Heterologous Nucleic Acid Sequence: a nucleic acid sequence not naturally associated with the host genome into which it is introduced, including non-naturally occurring multiple copies of a naturally occurring nucleic acid sequence.

Homologous Nucleic Acid Sequence: a nucleic acid sequence naturally associated with a host genome into which it is introduced.

Homologous Recombination: the reciprocal exchange of nucleic acid fragments between homologous nucleic acid molecules.

Isolated: in the context of the present invention, an isolated nucleic acid molecule or an isolated enzyme is a nucleic acid molecule or enzyme that, by the hand of man, exists apart from its native environment and is therefore not a product of nature. An isolated nucleic acid molecule or enzyme may exist in a purified form or may exist in a non-native environment such as, for example, a transgenic host cell.

Minimal Promoter: promoter elements that are inactive or that have greatly reduced promoter activity in the absence of upstream activation. In the presence of a suitable transcription factor, the minimal promoter functions to permit transcription.

Nucleic Acid Molecule / Nucleic Acid Sequence: a linear segment of single- or double-stranded DNA or RNA that can be isolated from any source. In the context of the present invention, the nucleic acid molecule is preferably a segment of DNA.

Plant any plant at any stage of development, particularly a seed plant.

Plant Cell: a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.

Plant Cell Culture: cultures of plant units such as, for example, protoplasts, cell culture cells, cells in plant tissues, pollen, pollen tubes, ovules, embryo sacs, zygotes and embryos at various stages of development.

Plant material: leaves, stems; roots; flowers or flower parts, fruits, pollen, egg cells, zygotes, seeds, cuttings, cell or tissue cultures, or any other part or product of a plant.

Plant Organ: a distinct and visibly structured and differentiated part of a plant such as a root, stem, leaf, flower bud, or embryo.

Plant tissue: as used herein means a group of plant cells organized into a structural and functional unit Any tissue of a plant *in planta* or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

Promoter an untranslated DNA sequence upstream of the coding region that contains the binding site for RNA polymerase II and initiates transcription of the DNA. The promoter region may also include other elements that act as regulators of gene expression.

Protoplast an isolated plant cell without a cell wall or with only parts of the cell wall.

Regulatory Sequence: an untranslated nucleic acid sequence that assists in, enhances, or otherwise affects the transcription, translation or expression of an associated structural nucleic acid sequence that codes for a protein or other gene product. Regulatory sequences include promoters. A promoter sequence is usually located at the 5' end of a translated sequence, typically between 20 and 100 nucleotides from the 5' end of the translation start site. Regulatory sequences may also include transcribed but untranslated nucleic acid sequences located 5' and 3' to coding sequences. These untranslated RNA's are typically involved in post-transcriptional regulation of gene expression.

Substantially Similar: with respect to nucleic acids, a nucleic acid molecule that has at least 60 percent sequence identity with a reference nucleic acid molecule. In a preferred embodiment, a substantially similar DNA sequence is at least 80% identical to a reference DNA sequence; in a more preferred embodiment, a substantially similar DNA sequence is at least 90% identical to a reference DNA sequence; and in a most preferred embodiment, a substantially similar DNA sequence is at least 95% identical to a reference DNA sequence. A substantially similar nucleotide sequence typically hybridizes to a reference nucleic acid molecule, or fragments thereof, under the following conditions: hybridization at 7% sodium dodecyl sulfate (SDS), 0.5 M NaPO₄ pH 7.0, 1 mM EDTA at 50°C; wash with 2X SSC, 1% SDS, at 50°C. With respect to proteins or peptides, a substantially similar amino acid sequence is an amino acid sequence that is at least 90% identical to the amino acid sequence of a reference protein or peptide and has substantially the same activity as the reference protein or peptide.

Tolerance: the ability to continue normal growth or function when exposed to an inhibitor or herbicide.

Transformation: a process for introducing heterologous DNA into a cell, tissue, or plant, including a plant plastid. Transformed cells, tissues, or plants are understood to encompass not only the end product of a transformation process, but also transgenic progeny thereof.

Transformed / Transgenic / Recombinant: refer to a host organism such as a bacterium or a plant into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, or plants are understood to encompass not only the end product of a transformation process, but also transgenic progeny thereof. A "non-transformed", "non-transgenic", or "non-recombinant" host refers to a wild-type organism, e.g., a bacterium or plant, which does not contain the heterologous nucleic acid molecule.

Nucleotides are indicated by their bases by the following standard abbreviations: adenine (A), cytosine (C), thymine (T), and guanine (G). Amino acids are likewise indicated by the following standard abbreviations: alanine (Ala; A), arginine (Arg; R), asparagine (Asn; N), aspartic acid (Asp; D), cysteine (Cys; C), glutamine (Gln; Q), glutamic acid (Glu; E), glycine (Gly; G), histidine (His; H), isoleucine (lle; l), leucine (Leu; L), lysine (Lys; K), methionine (Met; M), phenylalanine (Phe; F), proline (Pro; P), serine (Ser, S), threonine (Thr, T), tryptophan (Trp; W), tyrosine (Tyr, Y), and valine (Val; V). Furthermore, (Xaa; X) represents any amino acid.

The present disclosure provides the promoter region for the *clpP* gene from the *Arabidopsis thaliana* plastid genome that encodes a plant homologue of the Clp ATP-dependent protease. The disclosed promoter can be used to drive expression of coding sequences for selectable marker genes or any other genes of interest in the plastids of transgenic plants. The promoter is useful for constitutive expression of transgenes in both green and non-green plastids and is therefore particularly useful for plastid transformation in plants such as maize, in which selection of regenerable transformants requires selection in non-green tissues.

The *Arabidopsis clpP* promoter can be incorporated into plastid transformation vectors and transformed into plastids according to methods known in the art, particularly those described in the following: U.S. Patent Nos. 5,451,513, 5,545,817, 5,545,818, and 5,576,198; Intl. Application Nos. WO 95/16783, WO 97/32011, and WO 97/32977; and Svab *et al.* (1993) and McBride *et al.* (1994).

The present disclosure also describes a novel method for utilizing protein-coding regions of plastid genes to isolate novel intervening regulatory sequences, such as novel promoters or 3' or 5' UTR's. Such a method is exemplified by Applicant's technique for isolating the *Arabidopsis* plastid *clpP* promoter region and the *Arabidopsis* plastid 16S rRNA promoter region, as set forth in detail in the Examples below. Briefly, isolation of these promoter regions is facilitated by the chance that gene order in the *Arabidopsis* plastid genome is conserved relative to that of *Nicotiana tabacum,* for which the entire plastid genome sequence is known. In tobacco, *clpP* is present in divergent orientation from the *psbB* gene, the coding sequence of which is conserved among a number of plant species. Because only 445 base pairs separate the *psbB* start codon from the divergently oriented start codon of *clpP* in tobacco, the sequences of the protein coding regions of the divergent *clpP* and *psbB* genes are used to design primers for PCR that amplify the noncoding intergenic region between these genes. This region includes the promoters for *psbB* in one orientation and *clpP* in the other. An expressed sequence tag (EST) sequence from *Arabidopsis* is found in an EST database that appears to include a portion of the *clpP* coding sequence and 5' untranslated RNA (5'UTR). The sequence of this EST is used to design primers for PCR amplification of the *clpP* promoter based on the *Arabidopsis* DNA sequence encoding the putative start of the *clpP* protein. These primers were paired with ones designed to match the highly conserved DNA sequences around the *psbB* start codon. Using these primers, a DNA fragment of approximately 500 nucleotides, which includes the *Arabidopsis* plastid *clpP* promoter region, is amplified from total DNA of *Arabidopsis.* A DNA fragment that includes the *Arabidopsis* plastid 16S rRNA promoter region is amplified in a like manner.

Using the above method, one of ordinary skill in the art can use the protein-coding regions of two nearby plastid genes to isolate intervening untranslated sequences such as promoters and other regulatory sequences from the plastid genome of any plant. Preferably the two plastid genes are adjacent, in that there are no other transcribed sequences between the two nearby plastid genes; however, it is foreseeable that this method will work even if there is a small gene, such as a gene, encoding a tRNA, in the amplified region between the two nearby plastid genes. In a preferred embodiment, one of ordinary skill in the art can use the above method to isolate a plastid *clpP* promoter from the plastid genome of any plant. In another preferred embodiment, one of ordinary skill in the art can use the above method to isolate a plastid 16S rRNA promoter from the plastid genome of any plant

The present disclosure further describes a method of using novel plastid promoters, such as the *Arabidopsis* plastid *clpP* or 16S rRNA promoters, to improve plastid transformation efficiency by reducing undesired recombination between native DNA sequences in the plastid genome and exogenous DNA sequences contained in chimeric DNA fragments that are incorporated into plastid transformation vectors. It is known that even relatively short regions of homology between native DNA sequences in the plastid genome and exogenous DNA sequences will ultimately cause somatic recombination in plastid transformants. This biological property has even been used as a means for eliminating selectable markers from plastid transformants in chloroplasts of the green alga *Chlamydomonas* by flanking the selectable marker with identical repeated heterologous DNA sequences. Although neither the minimum size tract of homology required nor the precise degree of sequence identity within a particular homology tract sufficient for recombination has been identified, as little as 50-bp of homology to the plastid genome may be enough to induce recombination. These recombination events are visible in transgenic plants as pale sectors in leaves resulting from division of cells in which plastid genome - rearrangements have occurred. In extreme cases the result is nearly white leaves with small patches of green indicating recombination occurring in the majority of somatic cells and their lineage.

The essential features of non-recombinogenic regulatory sequences (such as promoters and 5' and 3' UTR's) include both the ability to function correctly to control heterologous gene expression in the plastids of a plant species of interest, as well as the lack of sufficient sequence identity to promote homologous plastid recombination. The latter property may be achieved either by using a heterologous regulatory sequence derived from the plastid genome of a different plant species, which has diverged in sequence to less than 85-90% identity, or by sufficiently mutating a native regulatory sequence derived from the plastid genome of the same plant species. In one embodiment this method involves using the *Arabidopsis clpP* promoter to direct transcription of genes of interest in the plastids of heterologous plant species such as tobacco, maize, rice, soybean, tomato, potato, or others. In another embodiment this method involves using the *Arabidopsis* 16S rRNA promoter described in the Examples to direct transcription of genes of interest in the plastids of heterologous plant species such as tobacco, maize, rice, soybean, tomato, potato, or others. In addition to higher plant plastid genes, useful heterologous promoters or 5' and 3' UTR's for non-recombinogenic regulation of plastid transgenes may also be derived from plastid genes of lower plants or algae, chromosomal genes of cyanobacteria, or genomes of viruses that infect plant or algal chloroplasts or cyanobacterial cells.

Selection of mutated native genes from the same plant, which are incapable of undesired recombination, is facilitated by random mutagenesis of regulatory sequences such that the sequence identity is reduced to at most 90% relative to the starting sequence. The pool of randomly mutated regulatory sequences is then selected for the subset that still is plastid-active (capable of normal functioning in plant plastids) by cloning each mutant upstream of a selectable marker gene that operates in the plastid then transforming the entire pool of chimeric DNA's into the plastids of wildtype plants. Only those mutated sequences still capable of functioning in plastids will result in expression of the selectable marker in the transgenic plants. Transgenic plants expressing the selectable marker are also assessed for somatic recombination by observing the frequency of leaf sectoring. The targeted region of the plastid genome of a transformed plant expressing the selectable marker and having a desirable frequency of leaf sectoring is then sequenced to determine which mutated regulatory sequence is present. This mutated sequence thus meets the criteria of controlling expression in a plastid of a gene of interest and having sufficient sequence divergence relative to native plastid DNA sequences to reduce the frequency of undesired recombination.

### EXAMPLES

The invention will be further described by reference to the following detailed examples. These examples are provided for purposes of illustration only, and are not intended to be limiting unless otherwise specified. Standard recombinant DNA and molecular cloning techniques used here are well known in the art and are described by Ausubel (ed.), *Current Protocols in Molecular Biology,* John Wiley and Sons, Inc. (1994); T. Maniatis, E. F. Fritsch and J. Sambrook, *Molecular Cloning: A Laboratory Manual,* Cold Spring Harbor laboratory, Cold Spring Harbor, NY (1989); and by T.J. Silhavy, M.L. Berman, and L.W. Enquist, *Experiments with Gene Fusions,* Cold Spring Harbor Laboratory, Cold Spring Harbor, NY (1984).

### Example 1: Isolation of the Arabidopsis clpP Promoter Region

Isolation of the *Arabidopsis clpP* promoter region is facilitated by the chance that gene order in the *Arabidopsis* plastid genome is conserved relative to that of *Nicotiana tabacum,* a plant for which the entire plastid genome sequence is known. In tobacco, *clpP* is present in divergent orientation from the *psbB* gene, which has been sequenced from a number of plant species and shown to be conserved in sequence. An alignment of the *psbB* sequences of tobacco, maize, wheat, and *Nicotiana acumina* indicates that the first eight amino acids are identically conserved, as are their DNA coding sequences. In tobacco only 445 base pairs separate the *psbB* start codon from the divergently oriented start codon of *clpP*.

In view of the above, the Applicant postulates that the sequences of the protein-coding regions of the divergent *clpP* and *psbB* genes can be used to design primers for PCR that could amplify the noncoding intergenic region between these genes. This region will, in theory, include the promoters for *psbB* in one orientation and *clpP* in the other. An expressed sequence tag (EST) sequence from *Arabidopsis* is found in the TIGR NHC AtEST database (http://www.tigr.org) that appears to include a portion of the *clpP* coding sequence and 5' untranslated RNA (5'UTR). Because the putative translation of this sequence is similar to the mature *clpP* of *E*. *coli,* and hence does not appear to include a plastid transit peptide, it is postulated that this EST (Seq ID# P_3982 from the TIGR NHC AtEST database) represents a portion of the plastid *clpP* message. However, because Shanklin *et al.* (1995) have suggested that nuclear-encoded *clpP* homologs might exist in *Arabidopsis,* the Applicant is wary of finding these instead of the genuine plastid-encoded gene. Because EST's by definition come from expressed messages, ESTP_3982 is not expected to include any of the untranscribed *clpP* promoter region.

The nucleotide sequence of ESTP_3982 is used to design primers for PCR amplification of the *clpP* promoter based on the *Arabidopsis* DNA sequence encoding the start of the *clpP* protein in this plant. These primers are paired with ones designed to match the highly conserved DNA sequences around the *psbB* start codon, which Applicant postulates are similarly conserved in *Arabidopsis.* The primers used are: and These successfully amplify a DNA fragment of approximately 500 nucleotides from total DNA of A. *thaliana* (cv "Landsburg erecta") using *Pfu* thermostable DNA polymerase. The blunt-ended DNA fragment is sequenced both directly (using the cloning primers) and subsequent to cloning into the EcoRV site of vector pGEM5Zf(-) to construct plasmid pPH146b. The nucleotide sequence of this approximately 500-bp PCR fragment is given in SEQ ID NO:1. Sequence analysis reveals 86% sequence identity to the tobacco *clpP* promoter region over a 200-bp region extending upstream of the *clpP* start codon. Thus, SEQ ID NO:1 includes the *Arabidopsis clpP* promoter region.

### Example 2: Preparation of a Chimeric Gene Containing the Arabidopsis clpP Promoter and Native clpP 5' Untranslated Sequence Fused to a GUS Reporter Gene and Tobacco Plastid rps16 Gene 3' Untranslated Sequence in a Plastid Transformation Vector

### I. Amplification of the Arabidopsis Plastid clpP Gene Promoter and Complete 5' Untranslated RNA (5' UTR).

DNA from plasmid pPH146b is used as the template for PCR with a left-to-right "top strand" primer comprising an introduced EcoRl restriction site at position -234 relative to the ATG start codon of the Arabidopsis plastid *clpP* gene (nucleotide no. 263 of SEQ ID NO:1) (primer Aclp_P1a: 5'-GCGGAATTCATCATTCAGAAGCCCGTTCGT-3' (SEQ ID NO:4; EcoRl restriction site underlined)) and a right-to-left "bottom strand" primer homologous to the region from -21 to -1 relative to the ATG start codon of the *clpP* promoter that incorporates an introduced BspHI restriction site at the start of translation (primer Aclp_P2b: 5'-GCGTCATGAAATGAAAGAAAAAGAGAAT-3' (SEQ ID NO:5; BspHl restriction site underlined)). This PCR reaction is undertaken with *Pfu* thermostable DNA polymerase (Stratagene, La Jolla CA) in a Perkin Elmer Thermal Cycler 480 according to the manufacturer's recommendations (Perkin Elmer/Roche, Branchburg, NJ) as follows: 7 min 95°C, followed by 4 cycles of 1 min 95°C / 2 min 43°C / 1 min 72°C, then 25 cycles of 1 min 95°C / 2 min 55°C / 1 min 72°C. A 250 bp amplification product comprising the promoter and 5' untranslated region of the *Arabidopsis clpP* gene containing an EcoRl site at its left end and an BspHl site at its right end with two modifications near the ATG to correspond with the tobacco *clpP* sequence 5' UTR is gel purified using standard procedures and digested with EcoRl and BspHl (all restriction enzymes may be purchased from New England Biolabs, Beverly, MA).

### II. Amplification of the Tobacco Plastid rps16 Gene 3' Untranslated RNA Sequence (3'UTR).

Total DNA from *N. tabacum* c.v. "Xanthi NC" is used as the template for PCR as described above with a left-to-right "top strand" primer comprising an introduced Xbal restriction site immediately following the TAA stop codon of the plastid *rps16* gene encoding ribosomal protein S16 (primer rpsl6P_1a: 5'-GCGTCTAGATCAACCGAAATTCAATTAAGG-3' (SEQ ID NO:6; Xbal restriction site underlined)) and a right-to-left "bottom strand" primer homologous to the region from +134 to +151 relative to the TAA stop codon of *rps 16* that incorporates an introduced Hindill restriction site at the 3' end of the *rps16* 3' UTR (primer rps16P_1b: 5'-CGCAAGCTTCAATGGAAGCAATGATAA-3' (SEQ ID NO:7; HindIII restriction site underlined)). The amplification product comprising the 3' untranslated region of the *rps16* gene containing an Xbal site at its left end and a Hindlll site at its right end and containing the region corresponding to nucleotides 4943 to 5093 of the *N. tabacum* plastid DNA sequence (Shinozaki et al., 1986) is gel purified and digested with Xbal and Hindlll.

### III. Ligation of a GUS Reporter Gene Fragment to the clpP Gene Promoter and 5' and 3' UTR's.

An 1864 bp β-glucuronidase (GUS) reporter gene fragment derived from plasmid pRAJ275 (Clontech) containing an Ncol restriction site at the ATG start codon and an Xbal site following the native 3' UTR is produced by digestion with Ncol and Xbal. This fragment is ligated in a four-way reaction to the 250 bp EcoRl/BspHl *Arabidopsis clpP* promoter fragment, the 157 bp Xbal/Hindlll tobacco *rpsl6* 3'UTR fragment, and a 3148 bp EcoRl/Hindlll fragment from cloning vector pGEM3Zf(-) (Promega, Madison WI) to construct plasmid pPH165. Plastid transformation vector pPH166 is constructed by digesting plasmid pPRV111a (Zoubenko et al. 1994) with EcoRl and Hindlll and ligating the resulting 7287 bp fragment to a 2222 bp EcoRl/Hindill fragment of pPH165.

### Example 3: Isolation of the Arabidopsis 16S rRNA Gene Promoter Region

Isolation of the *Arabidopsis* 16S rRNA gene promoter region is facilitated by the chance that gene order in the *Arabidopsis* plastid genome is conserved relative to that of *Nicotiana tabacum,* a plant for which the entire plastid genome is known. In *Sinapis alba,* a closely related species to *Arabidopsis,* the 16S rRNA gene and valine tRNA are oriented as in tobacco (GenBank assession number CHSARRN1). The *Arabidopsis* 16S rRNA gene promoter region is isolated by PCR amplification (*PfuTurbo* DNA Polymerase, Stratagene, La Jolla, CA) using total *A. thaliana* (cv "Landsburg erecta") as template and the following primers that are conserved in both *Nicotiana* and *Sinapis alba:* "top strand" primer (5'-CAGTTCGAGCCTGATTATCC-3' (SEQ ID NO:8) and the "bottom strand" primer (5'-GTTCTTACGCGTTACTCACC-3' (SEQ ID NO:9). The predicted 379 bp (based on *Sinapis alba* sequence) amplification product comprising the *Arabidopsis* 16S rRNA gene promoter region corresponding to nucleotides 102508 to 102872 of the tobacco plastid genome (Shinozaki et al., 1986) is blunt end ligated into the *Eco*RV site of pGEM5Zf(-) (Promega) to construct pArab16S and sequence analysis and comparisons to the tobacco 16S rRNA promoter is performed. The *Arabidopsis* 16S rRNA gene promoter region product is 369 bp and is set forth as SEQ ID NO:10.

### Example 4: Preparation of a Chimeric Gene Containing the Arabidopsis 16S rRNA Gene

### Promoter and Native 5' Untranslated Sequence Fused to the Ribosome Binding Site of the Tobacco rbcL gene, a GUS Reporter Gene and the Tobacco Plastid rps16 Gene 3' Untranslated Sequence in a Plastid Transformation Vector

### I. Amplification of the Arabidopsis Plastid 16S rRNA Gene Promoter and Native 5' Untranslated Sequence (5' UTR) and Fusion to the Ribosome Binding Site of the Tobacco rbcL gene.

DNA from plasmid pArab16S is used as the template for PCR with a "top strand" primer comprising an introduced EcoRI restriction site at the 5' end of the 16S rRNA gene promoter region (position 63 of SEQ ID NO:10) (5'-GCCGGAATTCTCGCTGTGATCGAATAAGAATG-3' (SEQ ID NO:11; EcoRl restriction site underlined)). The "bottom strand" primer extends to position 172 (SEQ ID NO:10) of the 16S rRNA gene promoter 5' untranslated region, mutates three ATG's downstream of the transcription start site by changing position 151 (T to G) (SEQ ID NO:10), position 158 (A to C) (SEQ ID NO:10 and position 167 (A to C) (SEQ ID NO:10), fuses the ribosome binding site of the tobacco *rbcL* gene (positions 57569 to 57585) (Shinozaki et al., 1986) as a 5' extension to the 3' end of the 16S rRNA gene 5' UTR and introduces a BspHl site at the 3' end of the ribosome binding site (5'-GCCTTCATGAATCCCTCCCTACAACTATCCAGGCGCTTCAGATTCGCCTGGAGTT-3' (SEQ ID NO:12; BspHl restriction site underlined)). PCR amplification is performed with the *Pfu* Turbo DNA Polymerase kit (Stratagene). The 145 bp amplification product comprising the Arabidopsis 16S rRNA gene promoter and 5' untranslated region with three ATG's mutated and the ribosome binding site of the tobacco *rbcL* gene is gel purified and digested with EcoRl and BspHl, yielding a 131 bp product.

### II. Ligation of the Arabidopsis 16S rRNA Gene Promoter, 5' UTR and Ribosome Binding Site of the Tobacco rbcL gene to the GUS Reporte Gene and Tobacco Plastid rps 16 Gene 3' Untranslated Region (3' UTR) in a Plastid Transformation Vector.

An 1864 bp b-glucuronidase (GUS) reporter gene fragment derived from plasmid pRAJ275 (Clontech) containing an Ncol restriction site at the ATG start codon and an Xbal site following the stop codon is produced by digestion with Ncol and Xbal. This fragment is ligated in a four-way reaction to the 131 bp EcoRl/BspHl Arabidopsis 16S rRNA gene promoter, 5' UTR and tobacco *rbcL* ribosome binding site fragment, the Xbal/Hindlll tobacco *rps16* 3' UTR fragment described in Example 2, and a 3148 bp EcoRI/Hindlll fragment from cloning vector pGEM3Zf(-) (Promega, Madison, WI). A plastid transformation vector is constructed by digesting the previous construct with EcoRI and Hindlll and ligating the resulting 2.1 kb fragment to a 7.3 kb EcoRI/Hindlll fragment from plasmid pPRV111a (Zoubenko et al. 1994).

### Example 5: Biolistic Transformation of the Tobacco Plastid Genome

Seeds of *Nicotiana tabacum* c.v. 'Xanthi nc' are germinated seven per plate in a 1" circular array on T agar medium and bombarded 12-14 days after sowing with 1 µm tungsten particles (M10, Biorad, Hercules, CA) coated with DNA from the plasmids described above in Example 2 and Example 4essentially as described in Svab, Z. and Maliga, P. ((1993) *PNAS 90,* 913-917). Bombarded seedlings are incubated on T medium for two days after which leaves are excised and placed abaxial side up in bright light (350-500 µmol photons/m²/s) on plates of RMOP medium (Svab, Z., Hajdukiewicz, P. and Maliga, P. (1990) *PNAS* 87, 8526-8530) containing 500 µg/ml spectinomycin dihydrochloride (Sigma, St. Louis, MO). Resistant shoots appearing underneath the bleached leaves three to eight weeks after bombardment are subcloned onto the same selective medium, allowed to form callus, and secondary shoots are isolated and subcloned. Complete segregation of transformed plastid genome copies (homoplasmicity) in independent subclones is assessed by standard techniques of Southern blotting (Sambrook et al., (1989) *Molecular Cloning: A Laboratory Manual,* Cold Spring Harbor Laboratory, Cold Spring Harbor). BamHl/EcoRl-digested total cellular DNA (Mettler, I. J. (1987) *Plant Mol Biol Reporter* 5*,* 346-349) is separated on 1% Tris-borate (TBE) agarose gels, transferred to nylon membranes (Amersham) and probed with ³²P-labeled random primed DNA sequences corresponding to a 0.7 kb BamHI/HindIII DNA fragment from pC8 containing a portion of the *rps7*/*12* plastid targeting sequence. Homoplasmic shoots are rooted aseptically on spectinomycin-containing MS/IBA medium (McBride, K. E. et al. (1994) *PNAS* 91, 7301-7305) and transferred to the greenhouse.

## Claims

1. A plastid transformation method, comprising transforming a plastid of a host plant species with a chimeric gene comprising a regulatory sequence of an *Arabidopsis* plastid *clpP* gene operatively linked to a coding sequence of interest, wherein said regulatory sequence has a nucleotide sequence that is less than 90% identical to any native plastid *clpP* gene regulatory sequences in the host plant plastid, but still functions as a plastid-active regulatory sequence.

2. A method according to claim 1, wherein the regulatory sequence of the chimeric gene is obtained from the plastid genome of a different plant species to that of the plastid in the host plant.

3. A method according to claim 1, wherein the regulatory sequence of the chimeric gene is a mutated version of a regulatory sequence obtained from the plastid genome of the host plant.

4. A method according to claim 1, wherein the regulatory sequence is comprised within the sequence depicted in SEQ ID NO: 1.

5. A transgenic plant, plant cell, plant seed, plant tissue, or plant plastid, comprising a regulatory sequence of an *Arabidopsis* plastid *clpP* gene operatively linked to a coding sequence of interest, wherein said regulatory sequence has a nucleotide sequence that is less than 90% identical to any native plastid *clpP* gene regulatory sequence of the host plant, cell, seed, tissue or plastid, but still functions as a plastid-active regulatory sequence.

## Patentansprüche

1. Plastidentransformationsverfahren, das das Transformieren einer Plastide einer Wirtspflanzenart mit einem chimären Gen umfaßt, das eine regulatorische Sequenz eines clpP-Gens einer Arabidopsis-Plastide umfaßt, das funktionsfähig mit einer interessierenden codierenden Sequenz verbunden ist, worin die regulatorische Sequenz eine Nukleotidsequenz hat, die weniger als 90 % identisch mit einer beliebigen der regulatorischen Sequenzen des clpP-Gens der nativen Plastide in der Wirtspflanzenplastide ist, aber noch als Plastiden-aktive regulatorische Sequenz funktioniert.

2. Verfahren gemäß Anspruch 1, worin die regulatorische Sequenz des chimären Gens aus dem Plastidengenom einer unterschiedlichen Pflanzenart gegenüber derjenigen der Plastide in der Wirtspflanze erhalten wird.

3. Verfahren gemäß Anspruch 1, worin die regulatorische Sequenz des chimären Gens eine mutierte Version einer regulatorischen Sequenz ist, die aus dem Plastidengenom der Wirtspflanze erhalten wird.

4. Verfahren gemäß Anspruch 1, worin die regulatorische Sequenz innerhalb der in SEQ ID NO: 1 dargestellten Sequenz umfaßt ist.

5. Transgene (s/r) Pflanze, Pflanzenzelle, Pflanzensamen, Pflanzengewebe oder Pflanzenplastide, umfassend eine regulatorische Sequenz eines clpP-Gens der Arabidopsis-Plastide, das funktionsfähig mit einer interessierenden codierenden Sequenz verbunden ist, worin die regulatorische Sequenz eine Nukleotidsequenz hat, die weniger als 90 % identisch mit einer beliebigen regulatorischen Sequenz des clpP-Gens der nativen Plastide der Wirtspflanze, der Zelle, des Samens, des Gewebes oder der Plastide ist, aber noch als Plastiden-aktive regulatorische Sequenz funktioniert.

## Revendications

1. Méthode de transformation de plastide, comprenant la transformation d'un plastide d'une espèce de plante hôte par un gène chimère comprenant une séquence de régulation d'un gène *clpP* de plastide *d'Arabidopsis* lié de façon opérationnelle à une séquence de codage d'intérêt, où ladite séquence de régulation a une séquence nucléotidique qui présente moins de 90 % d'identité par rapport aux séquences de régulation du gène *clpP* de plastide natives dans le plastide de la plante hôte, mais présente toujours l'activité fonctionnelle d'une séquence de régulation dans le plastide.

2. Méthode selon la revendication 1, dans laquelle la séquence de régulation du gène chimère est obtenue à partir du génome d'un plastide d'une espèce de plante différente de celle du plastide de la plante hôte.

3. Méthode selon la revendication 1, dans laquelle la séquence de régulation du gène chimère est une version mutée d'une séquence de régulation obtenue à partir du génome du plastide de la plante hôte.

4. Méthode selon la revendication 1, dans laquelle la séquence de régulation est comprise dans la séquence représentée dans SEQ ID n°: 1.

5. Plante, cellule de plante, semence de plante, tissu de plante ou plastide de plante transgéniques comprenant une séquence de régulation d'un gène *clpP* de plastide *d'Arabidopsis* lié de façon opérationnelle à une séquence de codage d'intérêt, où ladite séquence de régulation a une séquence nucléotidique qui présente moins de 90 % d'identité par rapport aux séquences de régulation du gène *clpP* de plastide natives de la plante, de la cellule, de la semence, du tissu ou du plastide hôte, mais présente toujours l'activité fonctionnelle d'une séquence de régulation dans le plastide.
